# FASCICULE DE BREVET EUROPEEN

(11) **EP 2 303 195 B1**
(45) Date de publication et mention de la délivrance du brevet: **18.01.2012**
(21) Numéro de dépôt: 09772692.1
(22) Date de dépôt: 03.06.2009
(51) Int. Cl.: A61F 2/38

(54) **PROTHESE TOTALE DE GENOU**
KNIEGELENKSTOTALPROTHESE
TOTAL KNEE PROSTHESIS

(30) Priorité: 06.06.2008 FR 0853766
(43) Date de publication de la demande: 06.04.2011
(73) Titulaire: Bercovy, Michel, 75015 Paris (FR); Bracy, David, Windsor, VIC 3181 (AU); Kerboul, Luc, 75006 Paris (FR)
(72) Inventeur: Bercovy, Michel, 75015 Paris (FR); Bracy, David, Windsor, VIC 3181 (AU)
(74) Mandataire: Blanchard, Eugène Gilles
(86) Numéro de dépôt international: PCT/FR2009/051052
(87) Numéro de publication internationale: WO 2010/001010

(56) Documents cités:
- EP-A- 0 522 822
- EP-A- 1 611 871
- WO-A-00/13616
- FR-A- 2 852 819
- US-A- 6 152 960

## Description

La présente invention concerne le domaine des prothèses articulaires, et plus particulièrement dans ce domaine particulier une nouvelle prothèse totale de genou.

Les prothèses de l'articulation du genou doivent être conçues pour respecter deux règles principales :
- tribologique : elles doivent subir un minimum d'usure en utilisation ;
- cinématique : le fonctionnement de la prothèse doit être le plus confortable possible pour le patient dans toutes les activités quotidiennes.

Et surtout la tribologie doit satisfaire à la cinématique et inversement la cinématique doit être conforme à la tribologie. C'est dans le cadre de cette double obligation réciproque que se situe la présente invention.

Dans ce domaine des prothèses de genou, de nombreuses propositions antérieures de prothèses totales de genou ont déjà été faîtes. Ces prothèses totales comportent traditionnellement trois éléments fonctionnels : un élément fémoral destiné à être implanté sur l'extrémité inférieure du fémur, un élément tibial adapté pour être implanté sur l'extrémité supérieure du tibia, et enfin un insert articulaire, généralement en polyéthylène, destiné à être inséré entre l'élément fémoral et l'élément tibial. L'insert articulaire est conformé pour autoriser et guider les mouvements de l'élément fémoral par rapport à l'élément tibial dans tous les mouvements de l'articulation prothétique en flexion et/ou rotation selon des cinématiques les plus proches possibles de celles du genou anatomique.

A cette fin, l'élément femoral comporte deux condyles, respectivement médial et latéral, délimitant entre eux une échancrure, dite intercondylienne et l'insert articulaire présente lui une surface supérieure comportant deux cavités glénoïdes dont les surfaces sont de forme sensiblement complémentaires des surfaces externes des condyles de l'implant fémoral et reliées entre elles par une surface de raccordement interglénoidienne saillante destinée à se loger dans l'échancrure intercondylienne.

Par ailleurs, l'insert articulaire présente également une surface inférieure configurée pour coopérer avec l'élément tibial. Ce dernier présente un plateau d'appui plan de l'insert dans lequel est formé un orifice sensiblement cylindrique et la surface inférieure de l'insert est quant à elle plane et comporte un pion de calage et de pivotement de l'insert sur l'élément tibial destiné à s'insérer dans l'orifice de l'élément tibial.

De façon connue en soit, la conformité ou congruence entre les surfaces de contact du composant fémoral et l'insert est un élément déterminant permettant la réduction de l'usure des surfaces articulaires, notamment de l'insert. De ce fait, les surfaces étant plus ou moins emboitées l'une par rapport à l'autre, une libération du mouvement se fait par le pivotement de l'insert sur le composant tibial autour d'un axe vertical, qu il soit central ou déporté latéralement.

De telles prothèses de genou sont traditionnellement dites à « plateau mobile »et la prothèse de la présente invention est préférentiellement de ce type.

Selon les patients à implanter, la prothèse peut enfin comporter un composant rotulien recouvrant la partie posterieure de la rotule et destiné à s'articuler avec le l'élément fémoral, dans sa partie trochléenne jusqu'à un angle de flexion d'environs 60°, puis partielement avec la partie condylienne au dela de cet angle de flexion.

Dans le but de permettre un mouvement de l'articulation plus ou moins physiologique plusieurs solutions ont été proposées dans l'état de la technique.

Une première de ces configurations consiste à forcer le déplacement de l'élément fémoral de la prothèse sur la surface supérieure de l'insert articulaire par l'intermédiaire d'une came formée par un pion ou une rampe situé entre les cavités glénoïdes de l'insert, cette came coopérant avec une butée transversale formée dans l'échancrure intercondylienne de l'élément fémoral. Ce type de prothèses est dit « postéro-stabilisé ».

Cette configuration de prothèse ne permet pas au genou prothétique de s'adapter à tous les mouvements car elle impose un déplacement postérieur constant ; de plus les mouvements de l'articulation en flexion-extension sont des mouvements contraints par la coopération de la came et de la butée, donc unidirectionnels et identiques en toutes circonstances et qui peuvent donc procurer des sensations de butée et de repoussement génantes, notamment au niveau rotulien.

Une autre configuration consiste à produire une prothèse de genou dite "à surfaces guidantes", c'est à dire dont seules la topographie des surfaces en contact des éléments fémoral et tibial et de l'insert de la prothèse participent à la résolution des impératifs physiologiques, tribologiques et de stabilité de l'articulation prothétique.

De telles prothèses à surfaces guidantes sont généralement dépourvues de toute surface de contact plane, d'arêtes saillantes et/ou d'élément de butée sur les surfaces articulaires de l'élément fémoral (condyles, échancrure intercondylienne, et trochlée fémorale) ainsi que sur la surface supérieure de l'insert articulaire (cavités glénoïdes et surface de raccordement interglénoïdienne).

Parmi ces dernieres, le document WO 00/23011 décrit une prothèse comportant une surface de contact entre insert et composant fémoral telle que le point de contact entre composant fémoral et insert soit décalé vers l'avant ou vers l'arriere dans chaque plan sagittal adjacent. Cette configuration procure des contacts ponctuels ou linéaires et non surfaciques entre élémént fémoral et insert de la prothèse, responsables de points de pression élevée, donc de fortes contraintes mécaniques ponctuelles ou tranchantes sur l'insert articulaire de la prothèse, accélérant l'usure de celle-ci.

Dans d'autres propositions telles que les demandes de brevet FR 2852819, et WO 2005/122967 le composant fémoral est posé dans une cuvette à la face supérieure de l'insert, dans laquelle il est maintenu par une paroi formée de toute la partie antérieure de insert qui empeche tout mouvement de rotation entre ces deux parties, sans générer un mouvement précis.

Enfin, d'autres propositions telle que celle décrite dans le document EP 1354371 A1, ont évité tout contact central entre le dôme de l'insert et l'échancrure intercondylienne du composant ce qui supprime l'effet de guidage central du dôme de l'insert et revient à une prothèse bicondylienne s'appuyant dans les deux glènes. Par ailleurs, dans ce type d'articulation prothétique du genou, les appuis condyliens médial et latéral dans les cavités glénoïdes de l'insert sont asymétriques, créant plusieurs source de problèmes. L'un d'eux réside dans le fait qu'un couple de descellement est créé par une compression dans la cavité médiale nettement supérieure à la compression dans la cavité latérale. De ce fait, l'élément tibial tend à se desceller du côté latéral (externe) du genou.

Par ailleurs, la plupart de ces prothèses possèdent une congruence satisfaisante dans les zones proches de l'extension, mais cette congruence diminue de façon importante lors de la flexion à cause de la diminution progressive des rayons de courbure condyliens, dans la partie postérieure de l'articulation, alors que le rayon de courbure du plateau tibial reste constant.

La présente invention a pour but de proposer une nouvelle prothèse totale de genou du type à plateau mobile et surfaces guidantes, qui ne présente pas les inconvénients précédemment cités des prothèses totales connues de l'art antérieur.

L'invention a tout particulièrement pour objectif d'améliorer le confort pour la personne porteuse de la prothèse, en favorisant une cinématique de fonctionnement de la prothèse non contrainte, qui remplisse au mieux tous les impératifs physiologiques, tribologiques et de stabilité de l'articulation prothétique.

Un autre objectif de la présente invention est également de fournir une nouvelle prothèse totale de genou qui prévienne les phénomènes dits de "roll-forward", c'est à dire de glissement antérieur de l'élément fémoral de la prothèse lors des mouvements de flexion, sans utilisation de butée ni de surface de came au niveau des surfaces articulaires de l'élément fémoral et de l'insert de la prothèse.

Pour répondre à ces objectifs, la présente invention propose une nouvelle prothèse totale de genou du type comportant:
- un implant fémoral comportant deux condyles délimitant entre eux une échancrure intercondylienne dont le profil externe est convexe dans un plan sagittal et concave dans un plant frontal et se raccorde dans tous les plans sans discontinuité avec le profil externe des condyles,
- un implant tibial comportant au moins un plateau d'appui destiné à reposer par sa surface inférieure sur l'extrémité du tibia, le cas échéant après résection, et
- un insert articulaire interposé entre l'implant tibial et l'implant fémoral et apte à coopérer avec eux par des surfaces de contact inférieure et supérieure de formes complémentaires respectivement de la surface supérieure du plateau d'appui de l'implant tibial et des profils externes des condyles et de l'échancrure intercondylienne de l'implant fémoral,
- la surface supérieure de l'insert articulaire comportant deux cavités glénoides de profil externe congruent du profil externe des condyles de l'implant fémoral et reliées entre elles par une surface de raccordement interglénoidienne saillante, de forme congruente de celle de l'échancrure intercondylienne, et de profil externe convexe dans un plan frontal et concave dans un plan sagittal et se raccordant dans tous les plans sans discontinuité avec le profil externe des cavités glénoides.

Selon la prothèse de l'invention et de façon caractéristique de celle-ci, l'échancrure intercondylienne et la surface de raccordement interglénoïdienne présentent des surfaces de contact congruentes qui définissent deux paraboloïdes hyperboliques complémentaires et en ce que la surface de contact des cavités glénoides de l'insert avec les condyles présente en section horizontale une forme ovoïde, dont le grand axe est oblique vers le haut et l'avant de l'insert et son point déclive est situé postérieurement à la ligne de plus grande largeur de l'insert quel que soit l'angle de flexion de l'implant femoral par rapport à l'implant tibial et l'insert.

Ainsi, on obtient trois situations mécaniques distinctes au cours des mouvements de l'articulation du genou :
1/ en position d'extension, un équilibre mécanique stable est obtenu par l'emboîtement complet des surfaces articulaires du composant fémoral dans l'insert, sur toute la largeur des cavités glenoides et de la surface interglénoidienne centrale de l'insert, entre ces cavités ;
2/ des lors que sous l'action des muscles le genou ne se trouve plus dans cette position d'extension, il se trouve dans une situation mécanique instable et se trouve guidé par effet de la gravité vers une position d'équilibre mécanique.
3/ en fin de flexion, le genou est en position d'équilibre par stabilisation des condyles fémoraux au niveau de puits gravitationels procurés dans chacune des cavités glénoïdes de l'insert et définis ci-après.

Selon une première caractéristique préférée de la prothèse de l'invention, les points les plus bas, dans deux plans sagittaux adjacents, de la surface de contact des cavités glénoïdes de l'insert sont tous situés sur une même droite dans un même plan transversal de l'insert.

Selon une autre caractéristique avantageuse, les surfaces de contact ovoïdes des cavités glénoïdes de insert sont reliées, à chaque degré de flexion par une surface isthmique concave en avant et en arrière et inscrite dans le paraboloïde hyperbolique définit par la surface de raccordement interglénoïdienne. Ceci autorise une transmission des contraintes dans la partie centrale de la prothèse, mais aussi une rotation axiale selon un axe vertical qui peut être central ou décalé vers l'une des glènes.

Toujours selon l'invention et un mode de réalisation préféré de celle-ci, la largeur de la surface de raccordement interglénoidienne, prise dans un plan horizontal, est décroissante de la partie antérieure vers la partie postérieure de l'insert. Cette caractéristique permet notamment de lutter efficacement contre les phénomènes de roll-forward de l'élément fémoral lors des mouvements de flexion.

Dans le même objectif, une autre caractéristique avantageuse de l'invention consiste en ce que la droite (Δ) passant par les points les plus hauts de la surface de raccordement interglénoïdienne présente une pente décroissante de la partie antérieure vers la partie postérieure de l'insert.

De préférence, cette droite (Δ) forme, dans un mode de réalisation préféré, un angle compris entre 1°et 15°, et de préférence encore compris entre 4° et 10°.

Toujours selon invention, la prothèse totale de genou comporte également un implant rotulien adapté pour coopérer par une surface articulaire avec l'implant fémoral, la surface articulaire dudit implant rotulien étant un paraboloïde hyperbolique complémentaire de celui définit par l'échancrure intercondylienne pour permettre un contact continu dans les plans sagittal et horizontal de l'implant rotulien et de l'implant fémoral depuis la partie la plus haute de la trochlée jusqu'à la partie postérieure des condyles dans toutes les positions de flexion, le contact se faisant sur toute la hauteur et toute la largeur de la face articulaire de la rotule.

Enfin, toujours selon l'invention, l'implant tibial de la prothèse de l'invention comporte sur une face inférieure des renforts latéraux de stabilisation. De façon avantageuse, ces renforts sont orientés vers l'aplomb du puit gravitationnel de l'insert, c'est-à-dire des points déclives de chacune des cavités glénoïdes de l'insert. De tels renforts latéraux permettent notamment de soulager les augmentations de contraintes qui ont tendance à créer un couple de déscellement de l'implant tibial lors des mouvements de flexion de l'articulation prothétique.

Diverses autres caractéristiques de la prothèse de la présente invention ressortiront à la lecture de la description détaillée qui va suivre, réalisée en référence aux figures annexées, qui montrent, à titre d'exemples non limitatifs, des formes de réalisation de la prothèse de l'invention. Parmi les figures annexées:
- la **figure 1** représente en perspective et en éclaté une prothèse totale de genou selon la présente invention, dans une forme préférée de réalisation,
- la **figure 2** représente une vue en coupe longitudinale médiane selon le plan II de la figure 1,
- la **figure 3** est une vue de dessus de l'insert articulaire de la prothèse représentée sur la figure 1,
- la **figure 4** est une vue en coupe longitudinale selon un plan II-II sagittal médian de implant fémoral et de l'insert articulaire de la prothèse de invention,
- la **figure 5** est une coupe dans un plan horizontal V-V de la figure 4, montrant les surfaces de contact ovoïdes entre les condyles fémoraux et les cavités glénoïdes de l'insert articulaire en position d'extension du genou prothétique formée par la prothèse de l'invention, ainsi que la zone de contact centrale isthmique reliant les deux cavités ovoides ;
- la **figure 6** représente une vue en coupe de l'implant fémoral et de l'insert de la prothèse de l'invention selon un plan frontal VI-VI sur la figure 4, comprenant l'axe de rotation du pion de l'insert,
- la **figure 7** représente une surface paraboloïde hyperbolique conforme aux surfaces de contact réciproque de l'échancrure intercondylienne et du dôme central de raccordement interglénoïdien de l'insert de la prothèse de l'invention.

Tel que cela ressort des figures, l'objet de l'invention concerne une prothèse totale **1** du genou comprenant classiquement un implant fémoral **2** et un implant tibial **3**, tout deux de préférence constitué d'un alliage métallique inoxydable biocompatible, et un insert articulaire **4**, généralement en une matière plastique tel que le polyéthylène. Cependant, un ou plusieurs éléments de cette prothèse peuvent également être constitués de céramique d'alumine ou d'alumine et de zircone, ou de résines biocompatibles par exemple.

L'implant fémoral **2** et l'implant tibial **3** sont tous deux destinés à être adaptés respectivement après réssection, sur l'épiphyse basse fémorale et l'épiphyse haute tibiale.

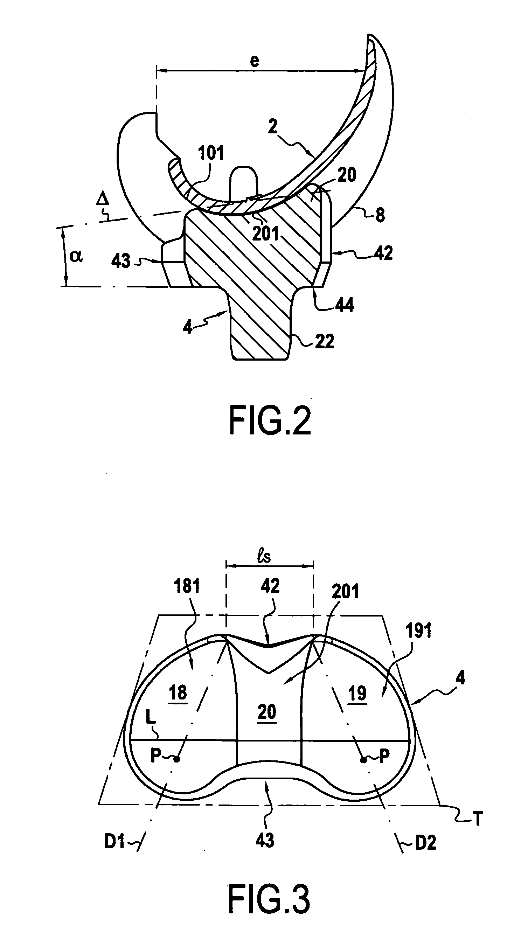

L'implant fémoral **2** présente vue de côté comme représenté partiellement sur la figure **2**, présente une forme sensiblement en U non symétrique entre les branches duquel est délimité un logement **5** de fixation sur l'épiphyse inférieure du fémur, en particulier par emboîtement de deux ergots saillants **6**.

L'implant fémoral **2** comporte également une face inférieure **7** articulaire réalisant deux condyles, respectivement un condyle médial **8** et un condyle latéral **9**, sépar rattachés l'un à l'autre par une échancrure intercondylienne **10**. Les deux condyles médial et latéral **8**, **9** de l'implant fémoral sont conformés de telle sorte que leur section, selon un plan sagittal, a la forme d'une spire, dont le rayon de curbure est décroissant de la partie extrême antérieure de l'implant formant la trochlée fémorale **11** vers la partie postérieure de l'implant.

Au niveau de la partie antérieure de l'implant fémoral **2**, la trochlée fémorale **11** s'étend ainsi dans le prolongement antérieur des deux condyles **8**, **9** et comporte deux joues de trochlée médiale **12** et latérale **13** prolongeant sans discontinuités le profil externe des condyles **8**, **9** respectivement médial et latéral. Les deux joues de trochlée **12**, **13** sont réunies par une gorge de trochlée **14** s'étendant elle-même sans discontinuités dans le prolongement antérieur de l'échancrure intercondylienne **10**. Ainsi la gorge de trochlée fémorale prolonge vers l'avant la forme en paraboloïde hyperbolique de l'échancrure intercondylienne sans aucune rupture de forme, de continuité ni d'angulation, depuis la partie la plus haute de la trochlée jusqu'à la partie la plus postérieure des condyles.

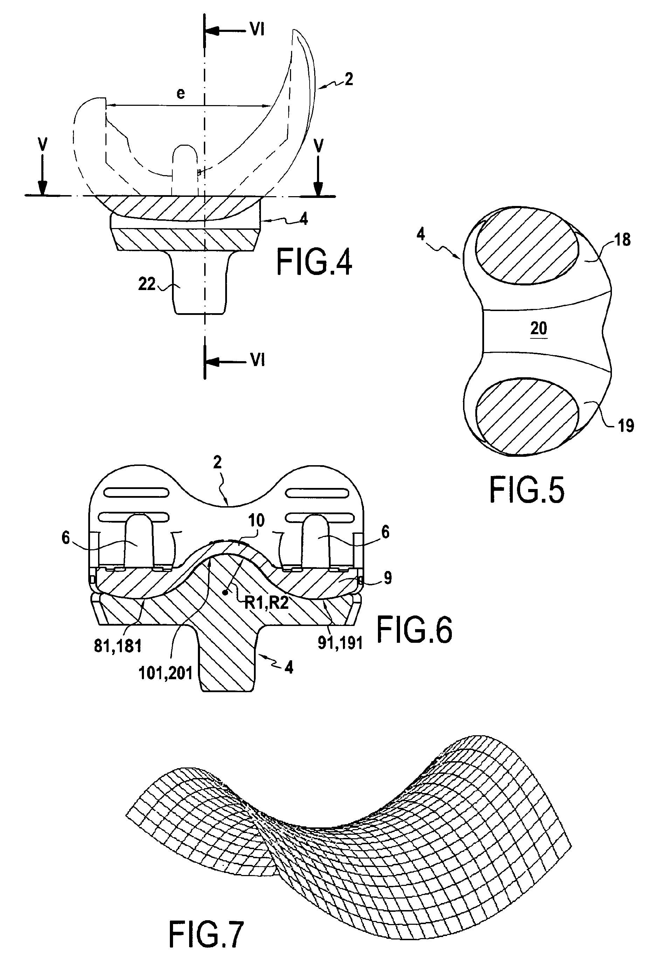

Comme cela est représenté sur le **figure 6**, chaque condyle **8**, **9** présente dans un plan frontal, c'est-à-dire transversalement au plan sagittal, un profil **81**, **91** convexe et arrondi. Entre les condyles, l'échancrure intercondylienne **10** présente quant à elle dans un plan frontal un profil **101** concave et arrondi, qui se raccorde sans discontinuités, ni arêtes avec celui des deux condyles **8**, **9** médial et latéral.

Ainsi, l'élément fémoral **2** est tel que, dans un plan frontal, le profil externe **101** de raccordement de l'échancrure intercondylienne **10** avec le profil externe **81**, **91** des condyles **8**, **9** présente un rayon de courbure **R1** constant de la partie antérieure vers la partie postérieure de implant fémoral **2**.

Conformément à la présente invention, le profil externe **101** de l'échancrure intercondylienne **10** est tel que celui-ci défini une surface de contact médiane de l'implant fémoral **2** en forme de paraboloïde hyperbolique, tel que représentée à la **figure 7** auquel se raccorde latéralement sans discontinuités ni arêtes les profils externes **81**, **91** convexes (dans un plan frontal) des condyles **8**, **9**.

De même, le profil surfacique **15** de la gorge de trochlée **14** présente également sur la partie antérieure de l'implant fémoral une forme de paraboloïde hyperbolique auquel se raccorde latéralement les profils convexes des joues **12**, **13** de trochlée médiale et latérale, dans le prolongement antérieur des condyles **8**, **9**.

De façon classique et connue en soi, l'implant fémoral **2** est destiné à coopérer avec l'insert articulaire **4** et plus particulièrement à se déplacer sur la surface supérieure **41** de celui-ci selon un mouvement de roulement-glissement des condyles **8**, **9** et de l'échancrure intercondylienne **10** sur des parties **18**, **19**, **20** de forme complémentaires formées sur la surface supérieure **41** de l'insert articulaire.

A cet effet, l'insert **4** de la prothèse selon l'invention comporte sur sa surface supérieure deux cavités glénoïdes **18**, **19**, respectivement une cavité médiale **18** et une cavité latérale **19**, séparées l'une de l'autre selon l'axe médian de l'insert par une surface de raccordement interglénoïdienne **20** formée par un dôme saillant intercalé entre les deux cavités glénoïdes **18**, **19**.

Dans un plan frontal, le profil externe **201** de raccordement de la surface interglénoidienne **20** avec le profil externe **181**, **191** des cavités glénoides **18**, **19** de l'insert articulaire présente un rayon de courbure **R2** qui est constant de la partie antérieure **42** vers la partie postérieure **43** de l'insert.

De préférence, le rayon de courbure **R2** du profil externe **201** de raccordement de la surface interglénoidienne **20** avec le profil externe **181**, **191** des cavités glénoides **18, 19** de l'insert articulaire **4** et le rayon de courbure **R1** du profil externe **101** de raccordement de l'échancrure intercondylienne **10** sont sensiblement égaux dans des plans frontaux communs de sorte que l'échancrure intercondylienne **10** constitue une empreinte de la surface interglénoidienne **20** entre les deux condyles **8**, **9** de l'implant fémoral. Cependant, il est également possible de prévoir un rayon de courbure **R1** qui soit variable, de préférence de façon décroissante, de la partie antérieure vers la partie postérieure de l'implant fémoral, tout comme par ailleurs le rayon **R2** de la surface de raccordement interglénoïdienne de l'insert.

Conformément à la présente invention, la surface interglénoïdienne **20** présente une surface **201** de contact congruente de la surface de contact **101** de l'échancrure intercondylienne **10** de l'implant fémoral **2** et qui définit plus particulièrement un second paraboloïde hyperbolique, complémentaire de celui défini par ladite échancrure **10**. Cette surface **201** s'inscrit, comme la surface **101** de l'échancrure intercondylienne, dans une surface paraboloïde hyperbolique telle que représentée à la **figure 7**.

De plus, toujours conformément à invention, la surface de contact **181**, **191** de chacune des cavités glénoides **18**, **19** de l'insert **4** est conformée de sorte que, en section horizontale, cette surface présente une forme ovoïde et que le point déclive, c'est-à-dire son point le plus bas de cette surface, forme un puit gravitionnel **P** situé dans le tiers postérieur de l'insert, en pratique en retrait de la ligne **L** de plus grande largeur de l'insert.

La configuration des surfaces articulaires de l'implant fémoral **2** et de t'insert articulaire **4** de la prothèse **1** de la présente invention permet ainsi de procurer à l'articulation prothétique en fonctionnement une cinématique et une stabilité particulièrement proche de celle de l'articulation anatomique.

Plus précisément, ces surfaces articulaires **81**, **91**, **101** et **181**, **191**, **201** procurent un équilibre instable entre l'implant fémoral **2** et l'insert articulaire **4** dès lors que le genou n'est plus en extension complète afin que l'oscillation du composant fémoral **2** se produise uniquement par un roulement dans le sens antéro-postérieur des condyles fémoraux **8**, **9** dans les cavités glénoïdes **18**, **19** par guidage des condyles sur les surfaces de contact ovoïdes **181**, **191** vers le puit gravitationnel **P** ménagé dans le tiers postérieur desdites cavités, en arrière de la partie la plus large de l'insert. Cette action est uniquement produite par l'action de la gravité sur l'élément fémoral en équilibre instable et ne comporte aucun effet de came.

Ainsi, lors des mouvements de flexion de l'articulation prothétique du genou selon la présente invention, le mouvement de roulement des condyles fémoraux **8**, **9** dans les cavités glénoïdes **18**, **19** de l'insert est parfaitement stabilisé par la congruence complète des surfaces en contact **81**, **91**, **101** et **181**, **191**, **201** de l'implant fémoral **2** et de l'insert **4** dans toutes les positions de flexion/extension.

Les cavités glénoïdes de l'insert de la prothèse **1** de l'invention sont également formées telles que les points les plus bas, dans deux plans sagittaux adjacents, de leur surface de contact **181**, **191** sont situés sur une même droite dans un même plan médian de l'insert. De plus, ces surfaces de contact ovoïdes des cavités glénoïdes de l'insert sont reliées à la surface **201** de la surface **20**, à chaque degré de flexion par une surface isthmique concave vers l'avant et l'arrière et inscrite dans le paraboloïde hyperbolique définit par la surface intergléndidienne.

Les deux cavités **18**, **19** médiale et latérale forment ainsi pour les condyles fémoraux **8**, **9** deux rampes de guidage ovoïdes orientées chacune suivant deux grands axes **D1**, **D2** divergents l'un de l'autre par rapport au plan sagittal médian de l'insert **4** depuis la face antérieure **42** vers la face postérieure **43** de celui-ci et inclinées de haut en bas vers le puit gravitationnel **P** de chacune d'elles.

Congruence parfaite des surfaces articulaires de l'implant fémoral **2** et de l'insert articulaire **4** et conformation guidantes des surfaces **181**, **191** de contacts ovoïdes des cavités glénoides **18**, **19** dudit insert vers des points d'équilibre naturels tels que sont les puits gravitationnels **P** dans le tiers postérieur desdites cavités procurent ainsi à la prothèse **1** de l'invention une cinématique non Contrainte et une stabilité adéquate très proche de celles de l'articulation anatomique.

Par ailleurs, la congruence parfaite de l'échancrure intercondylienne **10** et de la surface interglénoïdienne **20** dont les profils de surfaces définissent deux paraboloïdes hyperboliques complémentaires participent également à la stabilisation des mouvements de roulement antéro-postérieur de l'implant fémoral **2** sur l'insert **4** mais permet également un « roulis » médio-latéral (lift-off en anglais) permettant le respect du lift-off naturel de l'articulation du genou au cours duquel l'appui du condyle n'est pas seulement glénoïdien mais également central, favorisant une transmission centrale des contraintes et évitant ainsi de créer un couple de descellement de l'implant tibial par rapport à la surface osseuse du tibia.

De surcroît, la largeur **ls** de la surface interglénoidienne **20**, prise dans un plan horizontal, est avantageusement décroissante de la face antérieure **42** vers la face postérieure **43** de l'insert **4**, et, de plus, la surface interglénoïdienne **20** est telle que la droite Δ passant par les points les plus hauts du dôme interglénoïdien présente une pente décroissante de la face antérieure vers la face postérieure de l'insert et préférence, en formant un angle a avec la surface inférieure **44** de l'insert compris entre 4° et 10°.

Ces deux caractéristiques de la surface intergléndidienne **20** permettent notamment de lutter efficacement contre les phénomènes de roll-forward de l'élément fémoral **2** lors des mouvements de flexion du genou.

En effet, la configuration légèrement relevée mais surtout plus large de la surface interglénoidienne **20** de la face antérieure **42** de l'insert vers la face postérieure **43** participe également au guidage des condyles **8**, **9** vers le puit gravitationnel P des cavités glénoïdes **18**, **19** tout en s'opposant au glissement vers l'avant (roll-forward) de l'implant fémoral **2** sur l'insert **4** lors de la flexion, ce qui améliore encore la stabilité de l'articulation prothétique formée par la prothèse **1** de l'invention.

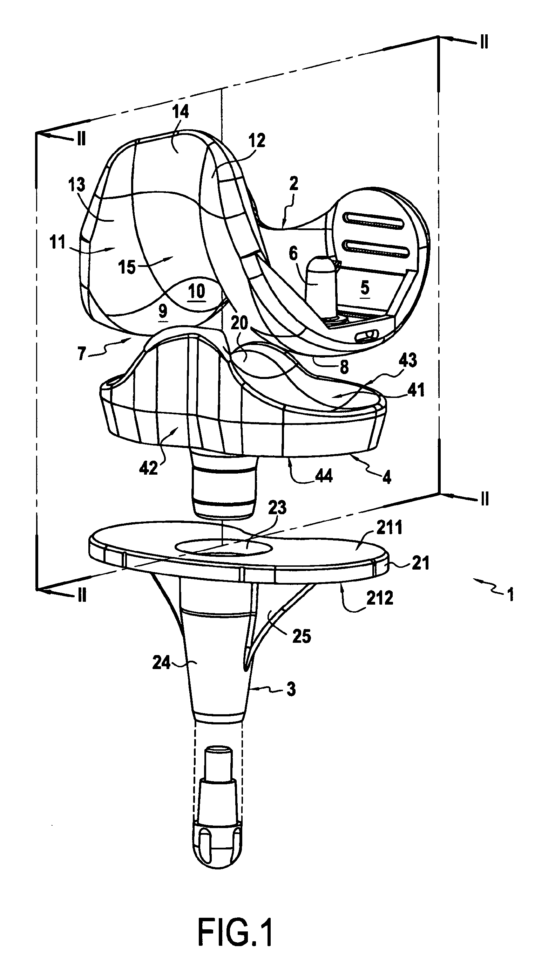

Comme cela est visible sur la **figure 1**, l'insert **4** prend appui par sa face inférieure **44** sur la surface supérieure **211** d'un plateau **21** de l'implant tibial **3** et est mobile en rotation sur ce plateau **21** autour d'un axe qui, en position d'utilisation de la prothèse, est disposé verticalement et est matérialisé par un pion **22** s'étendant en saillie depuis la surface inférieure **44** de l'insert **4**. Ce pion **22** est inséré dans un orifice **23** central percé dans le plateau **21** de l'implant tibial **3**, de façon connue en soi.

De préférence, la largeur li de l'insert **4**, prise dans une section horizontale, est décroissante depuis sa face postérieure **43** vers sa face antérieure **42**, ledit insert **4** s'inscrivant ainsi dans un trapèze **T**, comme représenté sur la **figure 3**. Ainsi l'insert **4** ne déborde t il pas, ou peu, des bords du plateau tibial **21** lors de ces rotations sur celui-ci, ce qui prévient l'endommagement des tissus conservés pour envelopper et stabiliser l'articulation prothétique.

L'implant tibial **3** quant à lui comporte également une tige médullaire **24** d'ancrage s'étendant depuis la face inférieure **212** du plateau **21** et destinée à venir reposer contre une surface épiphysaire réséquée du tibia (non représenté). De préférence comme représenté sur les figures, l'orifice **23** de réception du pion **22** de l'insert est percé et s'étend coaxialement à la tige d'ancrage **24**.

L'implant tibial **3** comporte également conformément à l'invention, des renforts latéraux **25** solidaires de la face inférieure **212** du plateau **21**. De façon avantageuse, ces renforts sont orientés vers l'aplomb des deux puits gravitationnels **P** des cavités glénoïdes **18**, **19** de l'insert **4**. De tels renforts latéraux permettent notamment de soulager les augmentations de contraintes qui ont tendance à créer un couple de déscellement de l'implant tibial **3** lors des mouvements de flexion de l'articulation prothétique.

De manière classique, la trochlée fémorale **11** de l'implant fémoral **2** est quant apte à coopérer avec la rotule naturelle ou avec un implant rotulien non représentée.

Dans le cadre de la présente invention, la prothèse totale **1** de genou proposée comporte également un implant rotulien adapté pour coopérer par une surface articulaire avec l'implant fémoral **2**. La surface articulaire dudit implant rotulien est en l'espèce également défini suivant un paraboloïde hyperbolique complémentaire de celui définit par la gorge de trochlée **14** et l'échancrure intercondylienne **10** de l'implant fémoral.

L'implant rotulien de la prothèse de la présente invention permet ainsi un contact continu dans les plans sagittaux et horizontaux avec l'implant fémoral depuis la partie la plus haute de la trochlée jusqu'à la partie postérieure des condyles dans toutes les positions de flexion, le contact se faisant sur toute la hauteur et toute la largeur de la face articulaire de la rotule.

Le glissement de l'implant rotulien ou, lorsqu'elle peut être conservée, de la rotule naturelle est parfaitement congruent avec un contact sur toute la surface de l'implant et la trochlée puis les condyles, avec contact continu sur toute la surface mediolatérale avec la trochlée et la zone intercondylienne de l'implant fémoral.

L'invention n'est pas limitée aux exemples décrits et représentés car diverses modifications peuvent y être apportées sans sortir de son cadre.

## Revendications

1. Prothèse totale de genou (**1**) comportant :
- un implant fémoral (**2**) comportant deux condyles (**8, 9**) délimitant entre eux une échancrure intercondylienne (**10**) dont le profil externe (**101**) est convexe dans un plan sagittal et concave dans un plan frontal et se raccorde dans tous les plans sans discontinuité avec le profil externe (**81, 91**) des condyles,
- un implant tibial (**3**) comportant au moins un plateau (**21**) d'appui destiné à reposer par sa surface inférieure (**212**) sur l'extrémité du tibia, le cas échéant après résection, et
- un insert articulaire (**4**) interposé entre avec l'implant tibial et l'implant fémoral et apte à coopérer avec eux par des surfaces de contact inférieure (**44**) et supérieure (**41**) de formes complémentaires respectivement de la surface supérieure (**211**) du plateau d'appui de l'implant tibial et des profils externes (**81, 91, 101**) des condyles et de l'échancrure intercondylienne de l'implant fémoral,
- la surface supérieure (**41**) de l'insert articulaire comportant deux cavités glénoides (**18, 19**) de profil externe (**181, 191**) congruent du profil externe (**81, 91**) des condyles (**7, 8**) de l'implant fémoral et reliées entre elles par une surface de raccordement initerglénoïdienne (**20**), de forme congruente de celle de l'échancrure intercondylienne (**10**), et de profil externe (**201**) convexe dans un plan frontal et concave dans un plan sagittal et se raccordant dans tous les plans sans discontinuité avec le profil externe (**181, 191**) des cavités glénoides,
l'échancrure intercondylienne (**10**) et la surface de raccordement interglénoïdienne (**20**) présentant des surfaces de contact (**101, 201**) congruentes **caractérisée en ce que** la surface de contact de l'échancrure intercondylienne et la surface de contact de la surface de raccordement interglénoïdienne s'inscrivent dans deux paraboloides hyperboliques complémentaires et **en ce que** la surface de contact (**181, 191**) des cavités glénoides (**18, 19**) présente en section horizontale une forme ovoïde et son point déclive (**P**) est situé postérieurement à la ligne de plus grande largeur (**L**) de l'insert.

2. Prothèse selon la revendication 1, **caractérisée en ce que** les points les plus bas, dans deux plans sagittaux adjacents, de la surface de contact (**181, 191**) des cavités glénoïdes (**18, 19**) de l'insert sont tous situés sur une même droite dans un même plan médian de l'insert.

3. Prothèse selon la revendication 1 ou la revendication 2, **caractérisée en ce que** les surfaces de contact (**181, 191**) ovoïdes des cavités glénoïdes de l'insert sont reliées, à chaque degré de flexion par une surface isthmique concave vers l'avant et vers l'arrière et inscrite dans le paraboloïde hyperbolique dans lequel s'inscrit la surface (**201**) de la surface de raccordement interglénoïdienne (**20**).

4. Prothèse selon l'une des revendications 1 à 3, **caractérisée en ce que** la largeur (**ls**) de la surface de raccordement interglénoidienne (**20**), prise dans un plan horizontal, est décroissante de la partie antérieure (**42**) vers la partie postérieure (**43**) de l'insert.

5. Prothèse selon l'une des revendications 1 à 4, **caractérisée en ce que** la droite (Δ) passant par les points les plus hauts de la surface de raccordement (**20**) présente une pente décroissante de la partie antérieure (**42**) vers la partie postérieure (**43**) de l'insert et **en ce que** cette droite forme un angle a compris entre 1°et 15°, et de préférence sensiblement compris entre 4° et 10°.

6. Prothèse selon l'une des revendications 1 à 5, **caractérisée en ce que** le rayon de courbure **R2** du profil externe (**201**) de raccordement de la surface de raccordement (**20**) interglénoidienne avec le profil externe (**181, 191**) des cavités glénoides (**18, 19**) de l'insert articulaire et le rayon de courbure **R1** du profil externe (**101**) de raccordement de l'échancrure intercondylienne (**10**) sont sensiblement égaux dans des plans frontaux communs de sorte que l'échancrure intercondylienne (**10**) constitue une empreinte de la surface de raccordement interglénoidienne (**20**) entre les deux condyles (**8, 9**) de l'implant fémoral (**2**).

7. Prothèse selon l'une des revendications 1 à 6, **caractérisée en ce que** la largeur (**li**) de l'insert, prise dans une section horizontale, est décroissante depuis sa partie postérieure **(43)** vers sa partie antérieure (**42**), ledit insert s inscrivant dans un trapèze.

8. Prothèse selon l'une des revendications 1 à 7, **caractérisée en ce qu'**elle comporte un implant rotulien adapté pour coopérer par une surface articulaire avec l'implant fémoral, la surface articulaire dudit implant rotulien étant un paraboloïde hyperbolique complémentaire de celui définit par l'échancrure intercondylienne.

9. Prothèse selon l'une des revendications 1 à 8, **caractérisée en ce que** l'implant tibial (**3**) comporte sur une face inférieure de son plateau (**21**) des renforts latéraux de stabilisation orientés vers l'aplomb de la zone d'appui des condyles dans les cavités glénoides de l'insert lorsque le genou est en position de flexion.

## Claims

1. A total knee prosthesis (**1**) comprising:
- a femoral implant (**2**) comprising two condyles (**8, 9**) delimiting between them an intercondylar notch (**10**), the external profile (**101**) of which being convex in a sagittal plane and concave in a frontal plane and being connected in all the planes without any discontinuity with the external profile (**81, 91**) of the condyles,
- a tibial implant (**3**) including at least one supporting plate (**21**) intended to rest through its lower surface (**212**) on the end of the tibia, if necessary after resection, and
- an articular insert (**4**) interposed between the tibial implant and the femoral implant and capable of co-operating with them through lower (**44**) and upper (**41**) contact surfaces with shapes respectively mating the upper surface (**211**) of the supporting plate of the tibial implant and the external profiles (**81, 91, 101**) of the condyles and the intercondylar notch of the femoral implant,
- the upper surface (**41**) of the articular insert including two glenoid cavities (**18, 19**) having an external profile (**181, 191**) being congruent with the external profile (**81, 91**) of the condyles (**8, 9**) of the femoral implant and connected together through an interglenoidal connecting surface (**20**), with a shape being congruent with the one of the intercondylar notch (**10**), and of an external profile (**201**) convex in a frontal plane and concave in a sagittal plane and connecting in all the planes without any discontinuity with the external profile (**181, 191**) of the glenoid cavities,
the intercondylar notch (**10**) and the interglenoidal connecting surface (**20**) presenting congruent contact surfaces (**101, 201**) **characterized in that** the contact surface of the intercondylar notch and the contact surface of the interglenoïdal connecting surface are inscribed in two mating hyperbolic paraboloids and **in that** the contact surface (**181, 191**) of the glenoid cavities (**18, 19**) has in a horizontal section an ovoid shape and has a lowest point (**P**) located posteriorly to the line of larger width (**L**) of the insert.

2. The prosthesis according to claim 1, **characterized in that** the lowest points, in two adjacent sagittal planes, of the contact surface (**181, 191**) of the glenoid cavities (**18, 19**) of the insert are all located on a same straight line in a same median plane of the insert.

3. The prosthesis according to claim 1 or claim 2, **characterized in that** the ovoid contact surfaces (**181, 191**) of the glenoid cavities of the insert are connected, at each flexural level through an isthmian surface concave towards the front and rear and inscribed in the hyperbolic paraboloid in which the surface (**201**) of the interglenoidal connecting surface (**20**) is inscribed.

4. The prosthesis according to any of claims 1 to 3, **characterized in that** the width (**ls**) of the interglenoidal connecting surface (**20**), taken in a horizontal plane, is decreasing from the anterior portion (**42**) towards the posterior portion (**43**) of the insert.

5. The prosthesis according to any of claims 1 to 4, **characterized in that** the straight line (Δ) passing through the highest points of the connecting surface (**20**) has a slope which decreases from the anterior portion (**42**) towards the posterior portion (**43**) of the insert and **in that** this straight line forms an angle a comprised between 1° and 15°, and preferably substantially comprised between 4° and 10°.

6. The prosthesis according to any of claims 1 to 5, **characterized in that** the radius of curvature **R2** of the external profile (**201**) for connecting the interglenoidal connecting surface (**20**) with the external profile (**181, 191**) of the glenoid cavities (**18, 19**) of the articular insert and the radius of curvature **R1** of the external profile (**101**) for connecting the intercondylar notch (**10**) are substantially equal in common frontal planes so that the intercondylar notch (**10**) forms an imprint of the interglenoidal connecting surface (**20**) between both condyles (**8, 9**) of the femoral implant (**2**).

7. The prosthesis according to any of claims 1 to 6, **characterized in that** the width (Ii) of the insert, taken in a horizontal section, is decreasing from its posterior portion **(43)** towards its anterior portion (**42**), said insert being inscribed in a trapezium.

8. The prosthesis according to any of claims 1 to 7, **characterized in that** it includes a patellar implant adapted for co-operating through an articular surface with the femoral implant, the articular surface of said patellar implant being a hyperbolic paraboloid mating the one defined by the intercondylar notch.

9. The prosthesis according to any of the claims 1 to 8, **characterized in that** the tibial implant (**3**) includes on its lower face of its plate (**21**), lateral stabilisation reinforcements oriented towards the base of the area supporting the condyles in the glenoid cavities of the insert when the knee is in a flexure position.

## Patentansprüche

1. Knietotalprothese (1) umfassend:
- ein Femurimplantat (2), das zwei Kondylen (8, 9) umfaßt, die zwischen sich eine interkondyläre Aussparung (10) begrenzen, deren Außenprofil (101) in einer Sagittalebene konvex und in einer Frontalebene konkav ist und in allen Ebenen ohne Unterbrechung an das Außenprofil (81, 91) der Kondylen anschließt,
- ein Tibiaimplantat (3), das wenigstens ein Stützplateau (21) umfaßt, welches dazu bestimmt ist, mit seiner Unterseite (212) auf dem Ende des Tibia aufzuliegen, gegebenenfalls nach Resektion, sowie
- einen Gelenkeinsatz (4), der zwischen dem Tibiaimplantat und dem Femurimplantat eingesetzt und geeignet ist, mit ihnen über eine untere Kontaktfläche (44) und eine obere Kontaktfläche (41) mit ergänzenden Formen zu der Oberseite (211) des Stützplateaus des Tibiaimplantats bzw. zu den Außenprofilen (81, 91, 101) der Kondylen und der interkondylären Aussparung des Femurimplantats zusammenzuwirken,
- wobei die Oberseite (41) des Gelenkeinsatzes zwei Gelenkpfannen (18, 19) aufweist, deren Außenprofil (181, 191) kongruent zu dem Außenprofil (81, 91) der Kondylen (8, 9) des Femurimplantats ist und die durch eine interglenoidale Verbindungsfläche (20) untereinander verbunden sind, deren Form kongruent zu der der interkondylären Aussparung (10) ist und deren Außenprofil (201) in einer Frontalebene konvex und in einer Sagittalebene konkav ist und in allen Ebenen ohne Unterbrechung an das Außenprofil (181, 191) der Gelenkpfannen anschließt,
wobei die interkondyläre Aussparung (10) und die interglenoidale Verbindungsfläche (20) kongruente Kontaktflächen (101, 201) aufweisen, **dadurch gekennzeichnet, daß** die Kontaktfläche der interkondylären Aussparung und die Kontaktfläche der interglenoidalen Verbindungsfläche in zwei ergänzenden hyperbolischen Paraboloiden liegen und daß die Kontaktfläche (181, 191) der Gelenkpfannen (18, 19) im horizontalen Querschnitt eine ovale Form aufweist und ihr abfallender Punkt (P) hinter der Linie größter Breite (L) des Einsatzes gelegen ist.

2. Prothese nach Anspruch 1, **dadurch gekennzeichnet, daß** die untersten Punkte - in zwei benachbarten Sagittalebenen - der Kontaktfläche (181, 191) der Gelenkpfannen (18, 19) des Einsatzes alle auf einer gleichen Geraden in einer gleichen Mittelebene des Einsatzes gelegen sind.

3. Prothese nach Anspruch 1 oder Anspruch 2, **dadurch gekennzeichnet, daß** die ovalen Kontaktflächen (181, 191) der Gelenkpfannen des Einsatzes bei jedem Beugungsgrad durch eine isthmische Fläche verbunden sind, die nach vorne und nach hinten konkav ist und in dem hyperbolischen Paraboloid liegt, in dem die Fläche (201) der interglenoidalen Verbindungsfläche (20) liegt.

4. Prothese nach einem der Ansprüche 1 bis 3, **dadurch gekennzeichnet, daß** die Breite (Is) der interglenoidalen Verbindungsfläche (20), in einer horizontalen Ebene gesehen, vom vorderen Teil (42) zum hinteren Teil (43) des Einsatzes abnehmend ist.

5. Prothese nach einem der Ansprüche 1 bis 4, **dadurch gekennzeichnet, daß** die Gerade (Δ), welche durch die obersten Punkte der Verbindungsfläche (20) verläuft, eine vom vorderen Teil (42) zum hinteren Teil (43) des Einsatzes abfallende Neigung aufweist und daß diese Gerade einen Winkel α im Bereich zwischen 1 ° und 15° und vorzugsweise im wesentlichen zwischen 4° und 10° bildet.

6. Prothese nach einem der Ansprüche 1 bis 5, **dadurch gekennzeichnet, daß** der Krümmungsradius R2 des Außenprofils (201) zum Verbinden der interglenoidalen Verbindungsfläche (20) mit dem Außenprofil (181, 191) der Gelenkpfannen (18, 19) des Gelenkeinsatzes und der Krümmungsradius R1 des Verbindungsaußenprofils (101) der interkondylären Aussparung (10) in gemeinsamen Frontalebenen im wesentlichen gleich sind, so daß die interkondyläre Aussparung (10) einen Eindruck der interglenoidalen Verbindungsfläche (20) zwischen den beiden Kondylen (8, 9) des Femurimplantats (2) bildet.

7. Prothese nach einem der Ansprüche 1 bis 6, **dadurch gekennzeichnet, daß** die Breite (li) des Einsatzes, in einem horizontalen Querschnitt gesehen, von seinem hinteren Teil (43) zu seinem vorderen Teil (42) abnehmend ist, wobei der Einsatz in einem Trapez enthalten ist.

8. Prothese nach einem der Ansprüche 1 bis 7, **dadurch gekennzeichnet, daß** sie ein Kniescheibenimplantat umfaßt, das geeignet ist, über eine Gelenkfläche mit dem Femurimplantat zusammenzuwirken, wobei die Gelenkfläche des Kniescheibenimplantats ein hyperbolisches Paraboloid ist, welches zu dem durch die interkondyläre Aussparung definierten komplementär ist.

9. Prothese nach einem der Ansprüche 1 bis 8, **dadurch gekennzeichnet, daß** das Tibiaimplantat (3) an einer Unterseite seines Plateaus (21) seitliche Stabilisierungsverstärkungen aufweist, die in lotrechter Richtung des Stützbereichs der Kondylen in den Gelenkpfannen des Einsatzes ausgerichtet sind, wenn das Knie sich in Beugeposition befindet.
